# EUROPEAN PATENT APPLICATION

(11) **EP 3 933 480 A1**
(43) Date of publication of application: **05.01.2022**
(21) Application number: 20779512.1
(22) Date of filing: 17.01.2020
(51) Int. Cl.: G02B 21/00, A61B 1/00, A61B 1/045, A61B 1/06, G02B 7/28, G02B 7/30, G02B 7/40, G02B 21/36, G02B 23/24, G03B 7/095, G03B 13/36, G03B 15/00, H04N 5/225, H04N 5/232, H04N 5/235

(54) **MEDICAL OBSERVATION SYSTEM**

(30) Priority: 25.03.2019 JP 2019057334
(71) Applicant: Sony Olympus Medical Solutions Inc., Hachioji-shi, Tokyo 192-0904 (JP)
(72) Inventor: KADO, Masataka, Hachioji-shi, Tokyo 192-0904 (JP)
(74) Representative: D Young & Co LLP
(86) International application number: PCT/JP2020/001572
(87) International publication number: WO 2020/195042

(57) **Abstract**

A medical observation system 1 is provided with an imaging unit 21 which captures an image of a subject to generate a captured image, a distance information acquiring unit which acquires subject distance information regarding subject distances from a specific position to corresponding positions on the subject that correspond to at least two pixel positions in the captured image, and an operation control section 264c which controls at least any of the focal position of the imaging unit 21, the brightness of the captured image, and the depth of field of the imaging unit 21 on the basis of the subject distance information.

## Description

### [Technical Field]

The present disclosure relates to a medical observation system.

### [Background Art]

Conventionally, a medical observation system using a surgical operation microscope that captures images of a predetermined visual field area of an observation target while magnifying the images has been known (see, PTL 1, for example).

The surgical operation microscope described in PTL 1 includes an imaging unit that captures an image of an observation target and a support that moves the imaging unit using movement with six degrees of freedom.

That is, the surgical operator grasps the imaging unit and uses the movement of six degrees of freedom of the support to position the imaging unit at a position facing the observation target of the patient lying on the operating table. The captured image taken by the imaging unit is magnified at a predetermined magnification and displayed on the display device. Then, the surgical operator executes the surgical operation while checking the captured image displayed on the display device.

### [Citation List]

### [Patent Literature]

[PTL 1]
Japanese Patent Laid-Open No. 2016-42981

### [Summary]

### [Technical Problem]

Incidentally, under the situation where the medical observation system is used, it is assumed that the operator's hand, surgical instrument, or the like may enter the angle of view of the imaging unit. In a case where an object brighter than the observation target enters the angle of view of the imaging unit in such a way, since it is considered that the brightness within the angle of view has become increased, the brightness of the captured image is adjusted so that the image to be observed becomes darker. That is, the captured image makes it difficult for the operator to recognize the observation target.

Therefore, there is demand for a technique capable of generating an image suitable for observation.

The present disclosure has been made in view of the above, and an object of the present disclosure is to provide a medical observation system capable of generating an image suitable for observation.

### [Solution to Problem]

In order to solve the above-mentioned problems and achieve the purpose, a medical observation system according to the present disclosure is provided with an imaging unit that captures an image of a subject and generates a captured image, a distance information acquiring unit configured to acquire subject distance information regarding a subject distance from a specific position to a corresponding position on the subject, the corresponding position corresponding to each of at least two pixel positions in the captured image, and an operation control section configured to control at least any of the focal position of the imaging unit, the brightness of the captured image, and the depth of field of the imaging unit on the basis of the subject distance information.

Further, in the medical observation system according to the present disclosure, the subject distance information includes depth map information obtained by detecting the subject distance from the specific position to the corresponding position on the subject, the corresponding position corresponding to a pixel position in the captured image, for each of the pixel positions, in the above disclosure.

Further, in the medical observation system according to the present disclosure, the operation control section determines a pixel position of the subject distance that is specific in the captured image, on the basis of the subject distance information, and controls the focal position of the imaging unit such that an area including the determined pixel position is in focus, in the above disclosure.

Further, in the medical observation system according to the present disclosure, the pixel position of the specific subject distance is a pixel position whose subject distance is the largest in the captured image, in the above disclosure.

Still further, in the medical observation system according to the present disclosure, the operation control section determines the pixel position of the specific subject distance in the captured image among the pixel positions in a central region including the center of the captured image, on the basis of the subject distance information, and controls the focal position of the imaging unit such that the area including the determined pixel position is in focus, in the above disclosure.

Still further, in the medical observation system according to the present disclosure, a detection region setting section configured to set a detection region in the captured image, and an evaluation value calculating section configured to calculate an evaluation value used for at least one of control of the focal position of the imaging unit and control of the brightness of the captured image executed by the operation control section on the basis of the image in the detection region in the captured image are further provided, and the detection region setting section determines a range of the subject distance to pay attention to, on the basis of the subject distance information, and sets an area including a pixel position of the subject distance included in the determined range of the subject distance in the captured image as the detection region, in the above disclosure.

In addition, in the medical observation system according to the present disclosure, a focal position detecting unit configured to detect the current focal position in the imaging unit is further provided, and the operation control section adjusts the brightness of the captured image on the basis of the subject distance information and the current focal position, in the above disclosure.

Moreover, in the medical observation system according to the present disclosure, a detection region setting section configured to set a detection region in the captured image, and an evaluation value calculating section configured to calculate an evaluation value used for controlling the brightness of the captured image by the operation control section, on the basis of the image in the detection region in the captured image are further provided, and the detection region setting section determines the currently observed region in the captured image on the basis of the subject distance information and the current focal position, and sets the determined region as the detection region, in the above disclosure.

Besides, in the medical observation system according to the present disclosure, a focal position detecting unit configured to detect a current focal position in the imaging unit is further provided, and the operation control section controls the depth of field of the imaging unit on the basis of the subject distance information and the current focal position.

Furthermore, in the medical observation system according to the present disclosure, an imaging unit includes an image pickup device that receives light from the subject and generates the captured image, and an aperture provided between the subject and the image pickup device and adjusting an amount of light incident on the image pickup device from the subject, and the operation control section controls the depth of field of the imaging unit by controlling the operation of the aperture, in the above disclosure.

Still further, in the medical observation system according to the present disclosure, an image processing unit configured to execute image processing on the captured image to adjust the depth of field is further provided, and the operation control section controls the depth of field of the imaging unit by controlling the operation of the image processing unit, in the above disclosure.

Still further, in the medical observation system according to the present disclosure, the operation control section determines the currently observed pixel position in the captured image on the basis of the subject distance information and the current focal position, and performs control such that the depth of field of the imaging unit is increased in a case where the subject distance at the determined pixel position is equal to or greater than a specific threshold value, in the above disclosure.

In addition, in the medical observation system according to the present disclosure, the distance information acquiring unit includes any of a phase difference sensor, a TOF (Time Of Flight) sensor, and a stereo camera, in the above disclosure.

In addition, in the medical observation system according to the present disclosure, the distance information acquiring unit is provided in the imaging unit, acquires the subject distance information, and generates the captured image, in the above disclosure.

### [Advantageous Effect of Invention]

According to the medical observation system related to the present disclosure, there is an effect of allowing an image suitable for observation to be generated.

### [Brief Description of Drawings]

[FIG. 1]

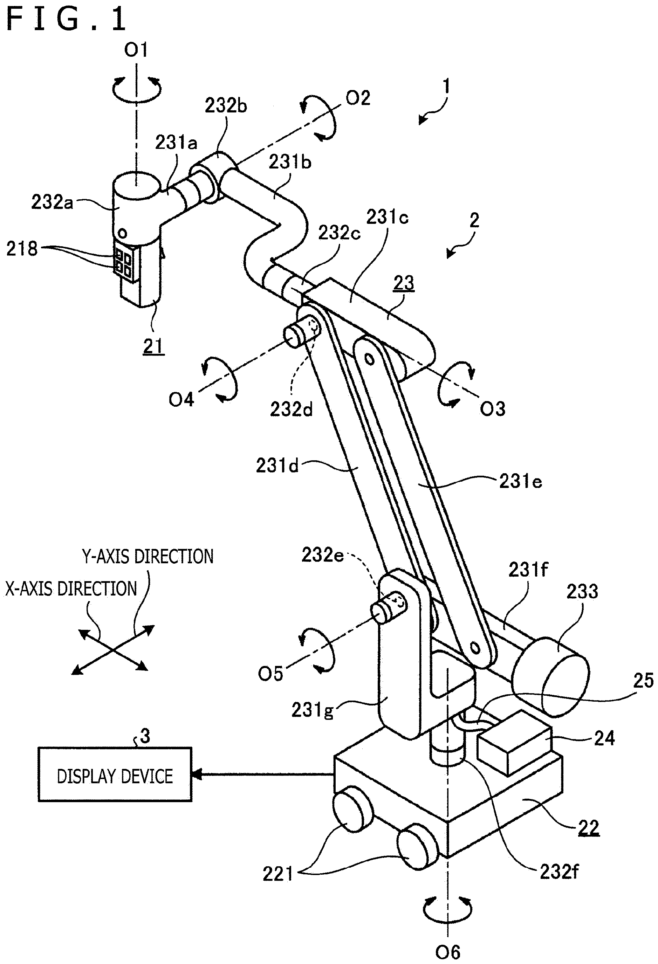

   FIG. 1 is a diagram illustrating a medical observation system according to a first embodiment.
[FIG. 2]

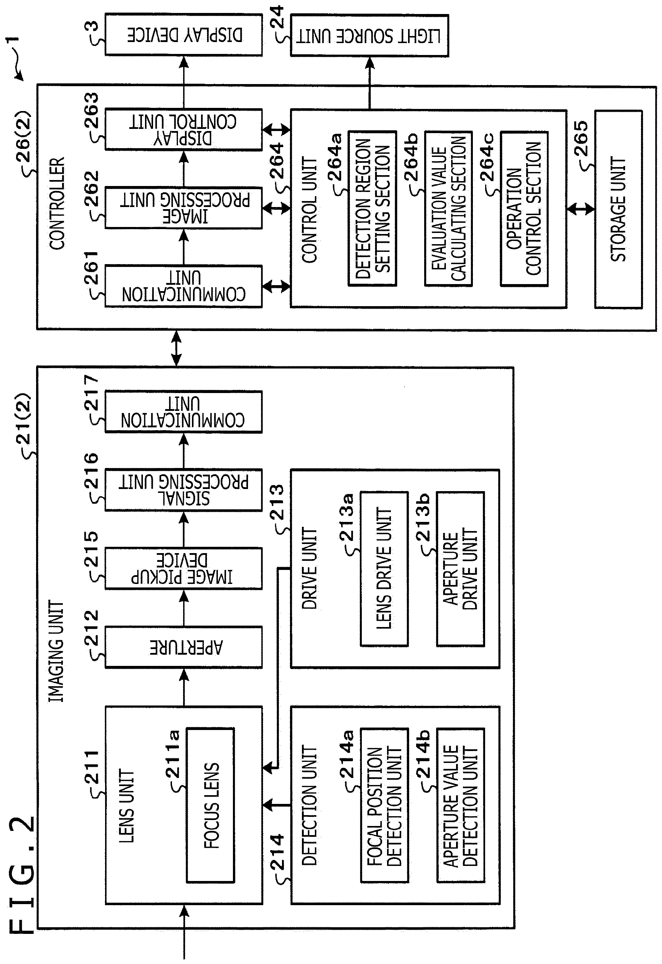

   FIG. 2 is a block diagram illustrating the medical observation system.
[FIG. 3]

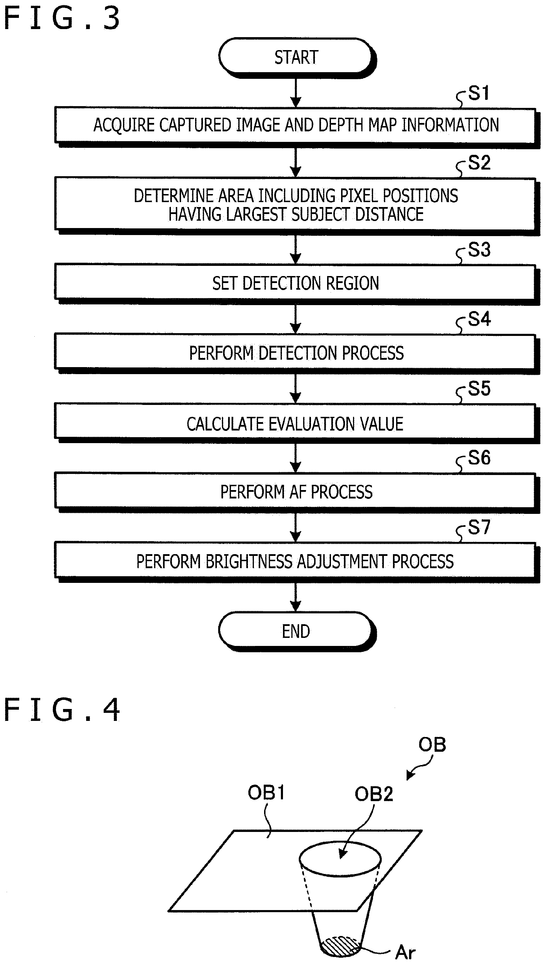

   FIG. 3 is a flowchart illustrating an operation of a controller.
[FIG. 4]
   FIG. 4 is a diagram illustrating the operation of the controller.
[FIG. 5]

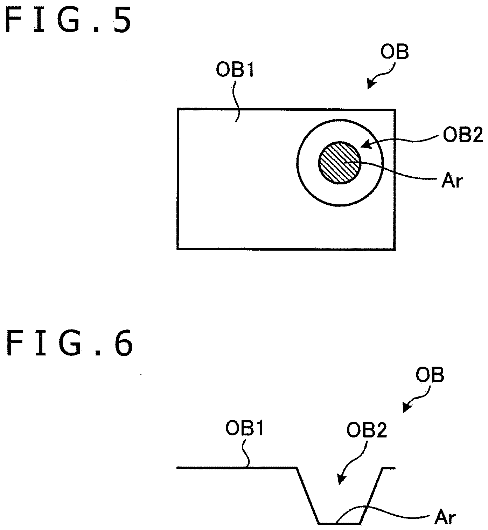

   FIG. 5 is a diagram illustrating the operation of the controller.
[FIG. 6]
   FIG. 6 is a diagram illustrating the operation of the controller.
[FIG. 7]

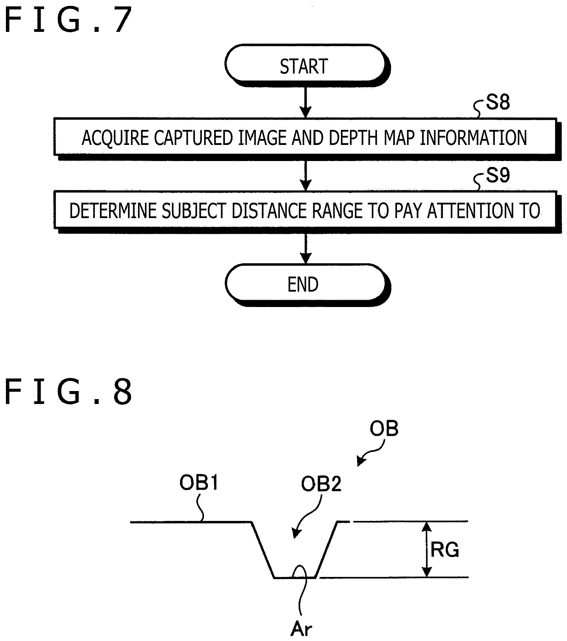

   FIG. 7 is a flowchart illustrating a pre-operation of a controller according to a second embodiment.
[FIG. 8]
   FIG. 8 is a diagram illustrating the pre-operation of the controller.
[FIG. 9]

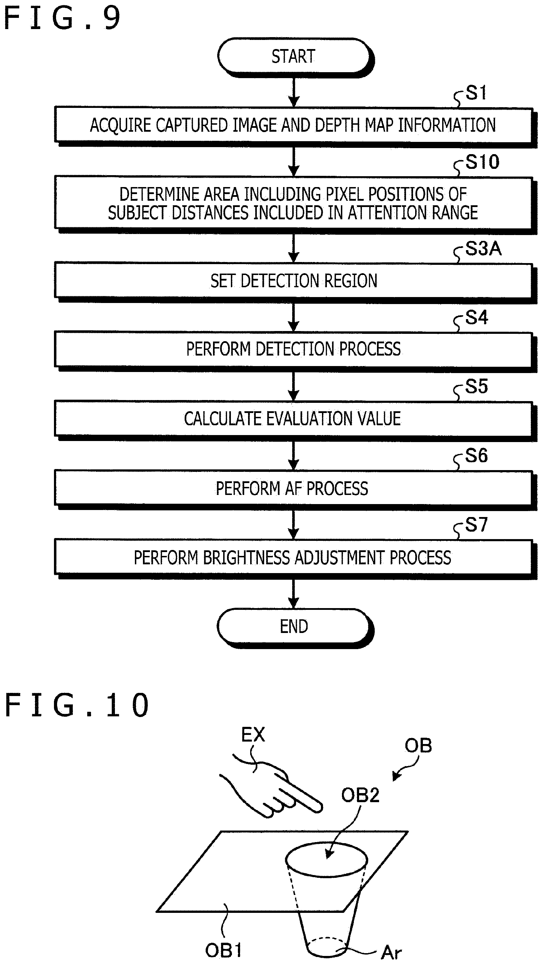

   FIG. 9 is a flowchart illustrating a main operation of the controller.
[FIG. 10]
   FIG. 10 is a diagram illustrating the main operation of the controller.
[FIG. 11]

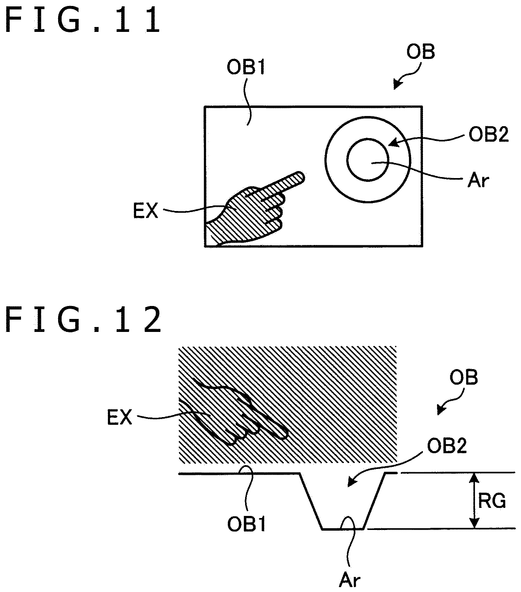

   FIG. 11 is a diagram illustrating the main operation of the controller.
[FIG. 12]
   FIG. 12 is a diagram illustrating the main operation of the controller.
[FIG. 13]

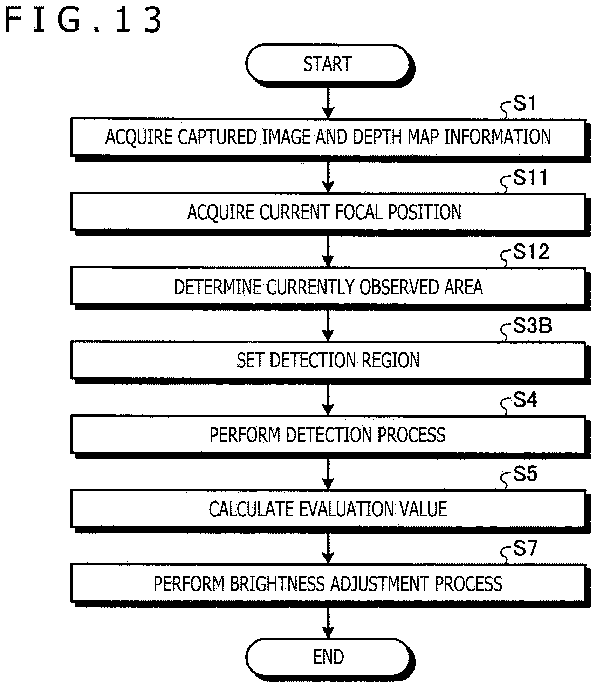

   FIG. 13 is a flowchart illustrating an operation of a controller according to a third embodiment.
[FIG. 14]

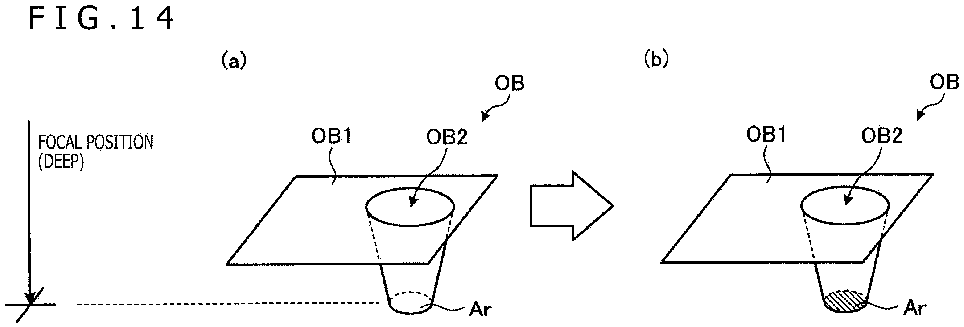

   FIG. 14 illustrates diagrams explaining the operation of the controller.
[FIG. 15]

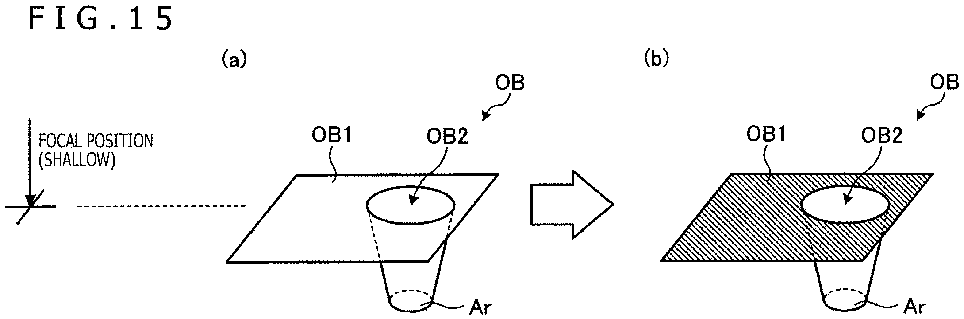

   FIG. 15 illustrates diagrams explaining the operation of the controller.
[FIG. 16]

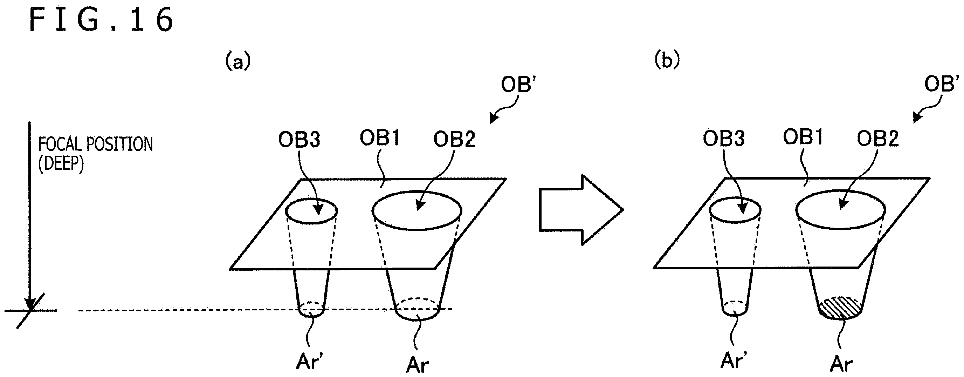

   FIG. 16 illustrates diagrams explaining a modification example of the third embodiment.
[FIG. 17]

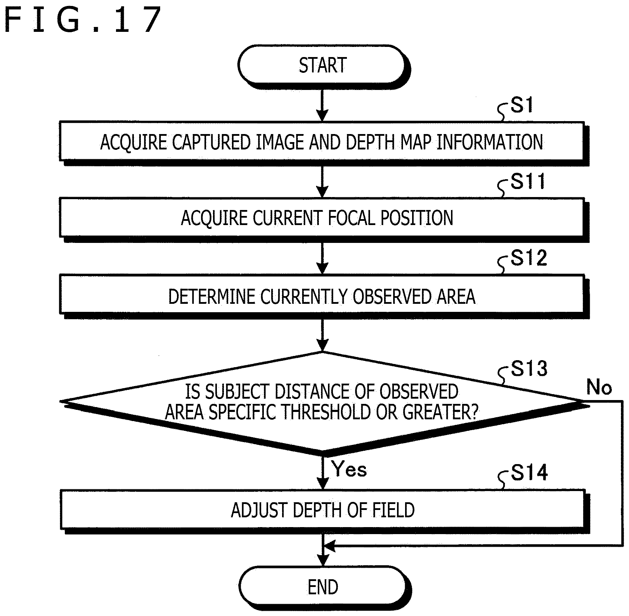

   FIG. 17 is a flowchart illustrating an operation of a controller according to a fourth embodiment.
[FIG. 18]

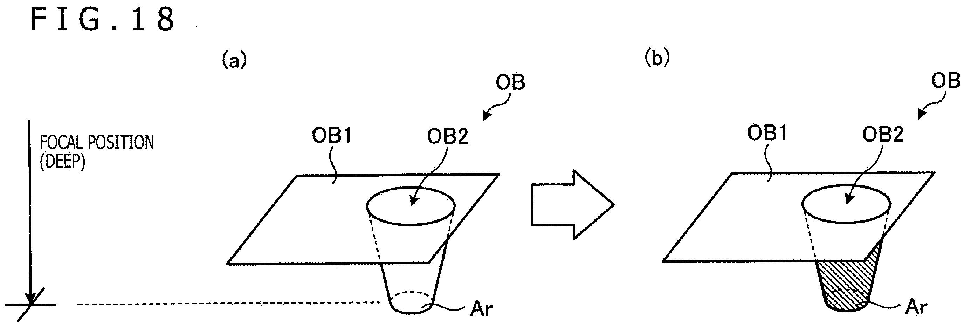

   FIG. 18 illustrates diagrams explaining the operation of the controller.
[FIG. 19]

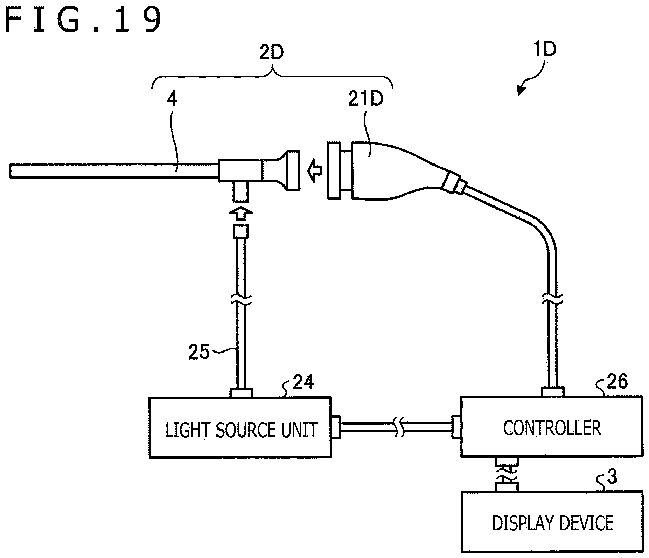

   FIG. 19 is a diagram illustrating a medical observation system according to a fifth embodiment.
[FIG. 20]

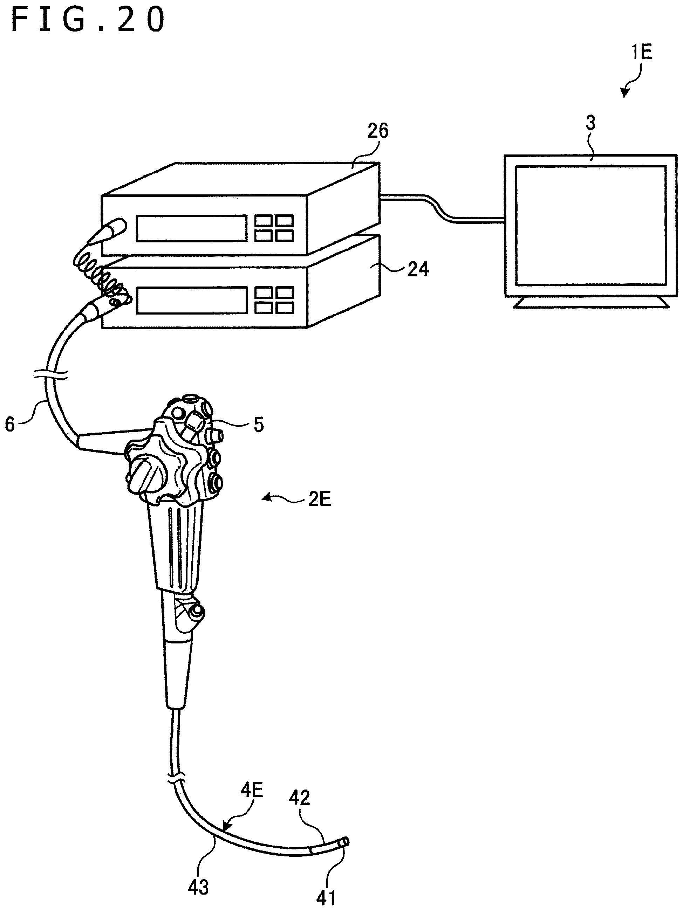

   FIG. 20 is a diagram illustrating a medical observation system according to a sixth embodiment.
[FIG. 21]

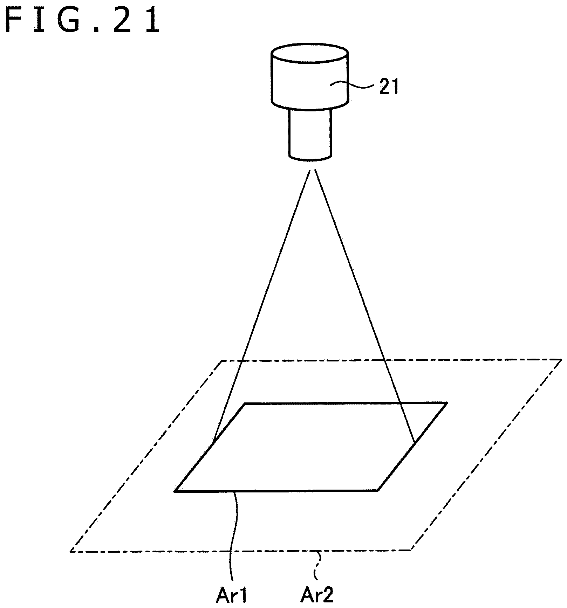

   FIG. 21 is a diagram illustrating a modification example for the first to fourth embodiments.

### [Description of Embodiments]

Hereinafter, embodiments for carrying out the present disclosure (hereinafter, referred to as embodiments) will be described with reference to the drawings. Note that the present disclosure is not limited to the embodiments described below. Further, in the description of the drawings, the same parts are designated by the same reference numerals.

### (First Embodiment)

### [Outline configuration of medical observation system]

FIG. 1 is a diagram illustrating a medical observation system 1 according to the first embodiment. FIG. 2 is a block diagram illustrating the medical observation system 1.

The medical observation system 1 is a system that captures images of an observation target (subject) and displays the captured image obtained by this image capturing in order to support microscopic surgical operations (microsurgery) such as neurosurgical operations or to perform endoscopic operations. As illustrated in FIG. 1 or 2, this medical observation system 1 is provided with a medical observation device 2 for capturing images of an observation target and a display device 3 that includes a display using a liquid crystal or an organic EL (Electro Luminescence) and displays the captured image obtained by the medical observation device 2.

The medical observation device 2 is a surgical operation microscope that captures images of a predetermined visual field area of an observation target while magnifying the images. As illustrated in FIG. 1 or 2, the medical observation device 2 includes an imaging unit 21, a base 22 (FIG. 1), a support 23 (FIG. 1), a light source unit 24, a light guide 25 (FIG. 1), and a controller 26 (FIG. 2).

As illustrated in FIG. 2, the imaging unit 21 includes a lens unit 211, an aperture 212, a drive unit 213, a detection unit 214, an image pickup device 215, a signal processing unit 216, and a communication unit 217.

The lens unit 211 includes a focus lens 211a (FIG. 2), captures a subject image from an observation target, and forms an image on the imaging surface of the image pickup device 215.

The focus lens 211a is configured by using a plurality of lenses and adjusts the focal position by moving along the optical axis.

Further, the lens unit 211 is provided with a focus mechanism (not illustrated) for moving the focus lens 211a along the optical axis.

The aperture 212 is provided between the lens unit 211 and the image pickup device 215 and adjusts the amount of light of the subject image from the lens unit 211 toward the image pickup device 215 under the control of the controller 26.

As illustrated in FIG. 2, the drive unit 213 includes a lens drive unit 213a and an aperture drive unit 213b.

In the AF process executed by the controller 26 and described later, the lens drive unit 213a operates the above-mentioned focus mechanism under the control of the controller 26 to adjust the focal position of the lens unit 211. Further, the lens drive unit 213a operates the above-mentioned focus mechanism and adjusts the focal position of the lens unit 211 in response to a user's operation by an operator such as a surgical operator on a focus switch 218 (FIG. 1) provided in the imaging unit 21.

The aperture drive unit 213b operates the aperture 212 under the control of the controller 26 to adjust the aperture value of the aperture 212.

As illustrated in FIG. 2, the detection unit 214 includes a focal position detection unit 214a and an aperture value detection unit 214b.

The focal position detection unit 214a includes a position sensor such as a photo interrupter and detects the position of the focus lens 211a (focal position) at the present time. Then, the focal position detection unit 214a outputs a signal corresponding to the detected focal position to the controller 26.

The aperture value detection unit 214b has a linear encoder or the like and detects the aperture value of the aperture 212 at the present time. Then, the aperture value detection unit 214b outputs a signal corresponding to the detected aperture value to the controller 26.

The image pickup device 215 has an image sensor that receives a subject image formed by the lens unit 211 and generates a captured image (analog signal). In the first embodiment, the image pickup device 215 is formed by integrating the image sensor with a TOF sensor (corresponding to a distance information acquiring unit according to the present disclosure) that acquires subject distance information (hereinafter referred to as depth map information) by the TOF method. The depth map information is obtained by detecting the subject distance from the position of the image pickup device 215 (corresponding to the specific position according to the present disclosure) to the corresponding position on the observation target, which corresponds to the pixel position in the captured image for each pixel position.

Incidentally, the distance information detecting unit according to the present disclosure is not limited to the TOF sensor, and a phase difference sensor, a stereo camera, or the like may be adopted.

Hereinafter, the depth map information and the captured image are collectively referred to as an image signal.

The signal processing unit 216 performs signal processing on the image signal (analog signal) from the image pickup device 215.

For example, the signal processing unit 216 performs signal processing such as processing to remove reset noise, processing to multiply the analog gain to amplify the analog signal, and A/D conversion on the image signal (analog signal) from the image pickup device 215.

The communication unit 217 is an interface that communicates with the controller 26, transmits an image signal (digital signal) subjected to signal processing by the signal processing unit 216 to the controller 26, and further receives the control signal from the controller 26.

The base 22 is a pedestal of the medical observation device 2 and is configured to be movable on the floor surface with casters 221 (FIG. 1).

The support 23 extends from the base 22 and holds the imaging unit 21 at the tip (end apart from the base 22). Then, the support 23 makes the imaging unit 21 three-dimensionally movable in response to an external force applied by the operator.

Incidentally in the first embodiment, the support 23 is configured to have six degrees of freedom for the movement of the imaging unit 21, but is not limited to this, and may have other different numbers of degrees of freedom.

As illustrated in FIG. 1, the support 23 includes first to seventh arms 231a to 231g and first to sixth joints 232a to 232f.

The first joint 232a is located at the tip of the support 23. The first joint 232a is fixedly supported by the first arm 231a and holds the imaging unit 21 rotatably around a first axis O1 (FIG. 1).

Here, the first axis O1 coincides with the observation optical axis of the imaging unit 21. That is, when the imaging unit 21 is rotated around the first axis O1, the orientation of the imaging field of view of the imaging unit 21 is changed.

The first arm 231a is a substantially rod-shaped member extending in a direction perpendicular to the first axis O1 and fixedly supports the first joint 232a at its tip.

The second joint 232b is fixedly supported by the second arm 231b and rotatably holds the first arm 231a around a second axis O2 (FIG. 1). Therefore, the second joint 232b makes the imaging unit 21 rotatable around the second axis O2.

Here, the second axis O2 is perpendicular to the first axis O1 and is parallel to the extending direction of the first arm 231a. That is, when the imaging unit 21 is rotated around the second axis O2, the direction of the optical axis of the imaging unit 21 with respect to the observation target is changed. In other words, the imaging field of view captured by the imaging unit 21 moves along the X axis (FIG. 1) perpendicular to the first and second axes O1 and O2 in the horizontal plane. Thus, the second joint 232b is a joint for moving the imaging field of view of the imaging unit 21 along the X axis.

The second arm 231b has a crank shape extending in a direction perpendicular to the first and second axes O1 and O2 and fixedly supports the second joint 232b at the tip.

The third joint 232c is fixedly supported by the third arm 231c and rotatably holds the second arm 231b around a third axis O3 (FIG. 1). Therefore, the third joint 232c makes the imaging unit 21 rotatable around the third axis O3.

Here, the third axis O3 is perpendicular to the first and second axes O1 and O2. That is, when the imaging unit 21 is rotated around the third axis O3, the direction of the optical axis of the imaging unit 21 with respect to the observation target is changed. In other words, the imaging field of view of the imaging unit 21 moves along the Y axis (FIG. 1) perpendicular to the X axis in the horizontal plane. Accordingly, the third joint 232c is a joint for moving the imaging field of view of the imaging unit 21 along the Y axis.

The third arm 231c is a substantially rod-shaped member extending in a direction substantially parallel to the third axis O3 and fixedly supports the third joint 232c at the tip.

The fourth joint 232d is fixedly supported by the fourth arm 231d and rotatably holds the third arm 231c around a fourth axis O4 (FIG. 1). Therefore, the fourth joint 232d makes the imaging unit 21 rotatable around the fourth axis O4.

Here, the fourth axis O4 is perpendicular to the third axis O3. That is, when the imaging unit 21 is rotated around the fourth axis O4, the height of the imaging unit 21 is adjusted. Thus, the fourth joint 232d is a joint for moving the imaging unit 21 in parallel.

The fourth arm 231d is a substantially rod-shaped member perpendicular to the fourth axis O4 and extending linearly toward the base 22 and fixedly supports the fourth joint 232d on one end side.

The fifth arm 231e has the same shape as the fourth arm 231d. Then, one end side of the fifth arm 231e is connected to the third arm 231c rotatably around an axis parallel to the fourth axis O4.

The sixth arm 231f has substantially the same shape as the third arm 231c. Then, the sixth arm 231f is connected to the other end side of each of the fourth and fifth arms 231d and 231e rotatably around axes parallel to the fourth axis O4, in a posture of a parallelogram formed with the third to fifth arms 231c to 231e. Further, a counterweight 233 (FIG. 1) is provided at the end of the sixth arm 231f.

The mass and the arrangement position of the counterweight 233 are adjusted so that the rotational moments generated around the fourth axis O4 and a rotational moment generated around a fifth axis O5 (FIG. 1) can be offset by the mass of each component provided on the tip side (the side where the imaging unit 21 is provided) of the support 23 with respect to the counterweight 233. That is, the support 23 is a balance arm (a configuration in which the counterweight 233 is provided). Incidentally, the support 23 may be configured such that the counterweight 233 is not provided.

The fifth joint 232e is fixedly supported by the seventh arm 231g and holds the fourth arm 231d rotatably around the fifth axis O5. Accordingly, the fifth joint 232e makes the imaging unit 21 rotatable around the fifth axis O5.

Here, the fifth axis O5 is parallel to the fourth axis O4. That is, when the imaging unit 21 is rotated around the fifth axis O5, the height of the imaging unit 21 is adjusted. Therefore, the fifth joint 232e is a joint for moving the imaging unit 21 in parallel.

The seventh arm 231g has a substantially L-shape including a first portion extending in the vertical direction and a second portion extending from a bend at a substantially right angle to the first portion and fixedly supports the fifth joint 232e on the first portion.

The sixth joint 232f rotatably holds the second portion of the seventh arm 231g around a sixth axis O6 (FIG. 1) while being fixedly supported by the base 22. Accordingly, the sixth joint 232f makes the imaging unit 21 rotatable around the sixth axis O6.

Here, the sixth axis O6 is an axis along the vertical direction. That is, the sixth joint 232f is a joint for moving the imaging unit 21 in parallel.

The first axis O1 described above is formed by a passive axis that allows the imaging unit 21 to passively rotate around the first axis O1 according to an external force applied by the operator, without using the power of an actuator or the like. Note that the second to sixth axes O2 to O6 are also similarly formed by passive axes, respectively.

One end of the light guide 25 is connected to the light source unit 24, which supplies the illumination light of the amount of light specified by the controller 26 to one end of the light guide 25.

One end of the light guide 25 is connected to the light source unit 24, and the other end is connected to the imaging unit 21. Then, the light guide 25 transmits, from one end to the other end, the light supplied from the light source unit 24 to supply the light to the imaging unit 21. The light supplied to the imaging unit 21 is emitted to the observation target from the imaging unit 21. The light emitted to the observation target and reflected by the observation target (subject image) is collected by the lens unit 211 in the imaging unit 21 and then captured by the image pickup device 215.

The controller 26 is provided inside the base 22 and comprehensively controls the operation of the medical observation system 1. As illustrated in FIG. 2, the controller 26 includes a communication unit 261, an image processing unit 262, a display control unit 263, a control unit 264, and a storage unit 265.

The communication unit 261 is an interface for communicating with the imaging unit 21 (communication section 217), receives an image signal (digital signal) from the imaging unit 21, and further transmits a control signal from the control unit 264.

Under the control of the control unit 264, the image processing unit 262 processes the captured image included in the image signal (digital signal) output from the imaging unit 21 and received by the communication unit 261.

For example, the image processing unit 262 multiplies the captured image (digital signal) by the digital gain that amplifies the digital signal. Further, the image processing unit 262 performs various types of image processing on the captured image after the multiplication of the digital gain, such as optical black subtraction processing, white balance (WB) adjustment processing, demosaic processing, color matrix calculation processing, gamma correction processing, YC conversion processing for generating luminance signals and color difference signals (Y, C_{B}/C_{R} signals).

Further, the image processing unit 262 executes the detection process on the basis of the captured image after execution of the various types of image processing described above.

For example, the image processing unit 262 executes the detection of contrast and frequency components of the image in the detection region, the detection of the luminance average value and the maximum and minimum pixels in the detection region by a filter or the like, the determination of the comparison with the threshold, and the detection of the histogram or the like (detection process) on the basis of pixel information (for example, a luminance signal (Y signal)) for each pixel in the detection region, which is at least a part of the entire image area of the captured image of one frame. Incidentally, the detection region is a region set by the control unit 264. Then, the image processing unit 262 outputs the detection information (contrast, frequency component, luminance average value, maximum/minimum pixel, histogram, etc.) obtained by the detection process to the control unit 264.

The display control unit 263 generates a video signal for display on the basis of the luminance signals and the color difference signals (Y, C_{B}/C_{R} signals) processed by the image processing unit 262 under the control of the control unit 264. Then, the display control unit 263 outputs the video signal to the display device 3. As a result, the display device 3 displays the captured image based on the video signal.

The control unit 264 includes a CPU (Central Processing Unit), an FPGA (Field-Programmable Gate Array), for example, and controls the operation of the entire controller 26 as well as the operation of the imaging unit 21, the light source unit 24, and the display device 3. As illustrated in FIG. 2, the control unit 264 includes a detection region setting section 264a, an evaluation value calculating section 264b, and an operation control section 264c.

Incidentally, the functions of the detection region setting section 264a, the evaluation value calculating section 264b, and the operation control section 264c will be described in "Operation of controller" to be described later.

The storage unit 265 stores a program executed by the control unit 264, information necessary for processing of the control unit 264, and the like.

### [Operation of controller]

Next, the operation of the controller 26 will be described.

FIG. 3 is a flowchart illustrating the operation of the controller 26. FIGS. 4 to 6 are views illustrating the operation of the controller 26. To be specific, FIG. 4 is a perspective view illustrating an observation target OB. FIG. 5 is a plan view of the observation target OB as viewed from above. FIG. 6 is a side view of the observation target OB.

Note that FIGS. 4 to 6 illustrate the observation target OB in which a recess OB2 is provided on a part of a surface OB1. Further, in FIGS. 4 and 5, the deepest area Ar of the recess OB2 is shaded.

First, the detection region setting section 264a acquires, via the communication unit 261, an image signal (captured image and depth map information) output from the imaging unit 21 after the image of the observation target OB is captured by the imaging unit 21 from above (step S1).

After step S1, the detection region setting section 264a determines the area including the pixel positions whose subject distances have the largest value in the entire image area of the captured image acquired in step S1 on the basis of the depth map information acquired in step S1 (step S2). The area including the pixel positions having the largest subject distance is a region corresponding to the deepest area Ar in the observation target OB.

After step S2, the detection region setting section 264a sets the area determined in step S2 as the detection region (step S3).

After step S3, the image processing unit 262 executes the detection process on the basis of the pixel information for each pixel of the detection region set in step S2 in the entire image area of the captured image acquired in step S1 (step S4). Then, the image processing unit 262 outputs the detection information obtained by the detection process to the control unit 264.

After step S4, the evaluation value calculating section 264b calculates the evaluation value on the basis of the detection information obtained by the detection process in step S4 (step S5).

To be specific, in step S5, the evaluation value calculating section 264b calculates the focusing evaluation value for evaluating the focusing state of the image in the detection region (area corresponding to the deepest area Ar) set in step S2 in the entire image area of the captured image acquired in step S1 on the basis of the detection information (contrast and frequency component). For example, the evaluation value calculating section 264b uses the contrast obtained by the detection process in step S4 or the sum of the high frequency components among the frequency components obtained by the detection process in step S4 as the focusing evaluation value. Note that the focusing evaluation value indicates a larger value as the subject is more focused on.

Further, in step S5, the evaluation value calculating section 264b calculates a brightness evaluation value to change the brightness of the image in the detection region (a region corresponding to the deepest area Ar) set in step S2 in the entire image area of the captured image to the reference brightness (change the detection information (luminance average value) to the reference luminance average value) on the basis of the detection information (luminance average value).

In the first embodiment, the evaluation value calculating section 264b calculates the first to fourth brightness evaluation values indicated below as the brightness evaluation values.

The first brightness evaluation value is the exposure time of each pixel in the image pickup device 215.

The second brightness evaluation value is the analog gain to be multiplied by the signal processing unit 216.

The third brightness evaluation value is the digital gain to be multiplied by the image processing unit 262.

The fourth brightness evaluation value is the amount of illumination light to be supplied by the light source unit 24.

After step S5, the operation control section 264c executes an AF process for adjusting the focal position of the lens unit 211 (step S6).

To be specific, in step S6, the operation control section 264c executes the AF process for positioning the focus lens 211a at the focal position so that the image in the detection region (the region corresponding to the deepest area Ar) set in step S2 in the entire image area of the captured image acquired in step S1 is in focus, by controlling the movement of the lens drive unit 213a, using a hill climbing method or the like on the basis of the focusing evaluation value calculated in step S5 and the current focal position detected by the focal position detection unit 214a.

After step S6, the operation control section 264c controls the operations of the image pickup device 215, the signal processing unit 216, the image processing unit 262, and the light source unit 24 so as to execute the brightness adjustment process for adjusting the brightness of the image in the detection region (the region corresponding to the deepest area Ar) set in step S2 in the entire image area of the captured image acquired in step S1 to the reference brightness (step S7) .

To be specific, the operation control section 264c outputs a control signal to the imaging unit 21 in step S7 and sets the exposure time of each pixel of the image pickup device 215 to the first brightness evaluation value calculated in step S5. Further, the operation control section 264c outputs a control signal to the imaging unit 21 and sets the analog gain to be multiplied by the signal processing unit 216 to the second brightness evaluation value calculated in step S5. Still further, the operation control section 264c outputs a control signal to the image processing unit 262 and sets the digital gain to be multiplied by the image processing unit 262 to the third brightness evaluation value calculated in step S5. In addition, the operation control section 264c outputs a control signal to the light source unit 24, and sets the amount of illumination light supplied by the light source unit 24 to the fourth brightness evaluation value calculated in step S5.

According to the first embodiment described above, the following effects are obtained.

Incidentally, in the observation target OB, the deepest area Ar is the region where the surgical operation is performed and is the region that the surgical operator wants to observe most.

The controller 26 according to the first embodiment sets a region corresponding to the deepest area Ar in the entire image area of the captured image as the detection region. Then, the controller 26 executes the AF process and the brightness adjustment process on the basis of the detection information obtained by the detection process in the detection region.

Accordingly, in the captured image, the deepest area Ar (the region that the operator wants to observe most) is automatically focused, and the brightness of the image corresponding to the deepest area Ar (the region that the operator wants to observe most) is automatically adjusted to a desired brightness. Therefore, according to the controller 26 related to the first embodiment, an image suitable for observation can be generated.

Further, in the first embodiment, the distance information acquiring unit according to the present disclosure is integrally mounted on the image pickup device 215 (imaging unit 21).

Therefore, the visual field area whose image is captured by the imaging unit 21 (see a visual field area Ar1 illustrated in FIG. 21, for example) and the depth map acquisition area where the distance information acquiring unit according to the present disclosure acquires the depth map information (see a depth map acquisition area Ar2 illustrated in FIG. 21, for example) can be the same area. That is, the process of matching the depth map acquisition area with the visual field area becomes unnecessary, and the processing load of the controller 26 can be reduced.

### (Modification Example of First Embodiment)

In the first embodiment described above, the controller 26 determines the pixel position of a specific subject distance (the pixel position having the largest subject distance) among the pixel positions of the entire image area of the captured image on the basis of the depth map information, but the method is not limited to this. For example, the controller 26 determines the pixel position of a specific subject distance (the pixel position having the largest subject distance) among the pixel positions in the central region including the center of the captured image on the basis of the depth map information. Then, the controller 26 sets the region including the determined pixel position as the detection region and executes the AF process so that the image in the detection region is in focus.

This modification is made by taking into consideration that the position of the imaging unit 21 is likely to be adjusted so that the position where the operation is performed is located in the central region of the captured image. That is, in the captured image, since the pixel position of a specific subject distance is determined only in the central region, an appropriate pixel position can be extracted while reducing the processing load of the controller 26.

### (Second Embodiment)

Next, the second embodiment will be described.

In the following description, similar components to those in the first embodiment will be designated by the same reference numerals, and detailed description thereof will be omitted or simplified.

In the second embodiment, the operation of the controller 26 is different from that of the first embodiment described above. The controller 26 according to the second embodiment executes the pre-operation and the main operation, respectively.

Hereinafter, the pre-operation and the main operation of the controller 26 will be described.

First, the pre-operation of the controller 26 will be described. The pre-operation is an operation to be performed in response to a user operation by the operator on an operation device (not illustrated) such as a mouse, keyboard, or touch panel provided on the controller 26 before performing a surgical operation on the observation target OB, for example.

FIG. 7 is a flowchart illustrating the pre-operation of the controller 26 according to the second embodiment. FIG. 8 is a diagram illustrating the pre-operation of the controller 26. Specifically, FIG. 8 is a side view of the observation target OB.

Incidentally, the observation target OB illustrated in FIG. 8 is the same observation target as the observation target OB illustrated in FIGS. 4 to 6.

First, the detection region setting section 264a acquires an image signal (captured image and depth map information) output from the imaging unit 21 after image of the observation target OB is captured by the imaging unit 21 from above, via the communication unit 261 (step S8). Here, in the captured image, only the observation target OB is the subject, and the operator's hand, surgical instrument, or the like is not included.

After step S8, the detection region setting section 264a determines the subject distance range to pay attention to (hereinafter referred to as the attention range) on the basis of the depth map information acquired in step S8 (step S9). In the example of FIG. 8, since only the observation target OB is included in the captured image, the detection region setting section 264a determines a range RG from the surface OB1 to the deepest area Ar in the observation target OB as the attention range. Then, the detection region setting section 264a stores the attention range determined in step S9 in the storage unit 265.

Next, the main operation of the controller 26 will be described. The main operation is an operation executed when a surgical operation is performed on the observation target OB, for example.

FIG. 9 is a flowchart illustrating the main operation of the controller 26. FIGS. 10 to 12 are diagrams illustrating the main operation of the controller 26. To be specific, FIG. 10 is a perspective view illustrating the observation target OB. FIG. 11 is a plan view of the observation target OB as viewed from above. FIG. 12 is a side view of the observation target OB.

Note that the observation target OB illustrated in FIGS. 10 to 12 is the same as the observation target OB illustrated in FIGS. 4 to 6.

In the main operation of the controller 26, the difference is that step S10 is adopted instead of step S2 with respect to the operation of the controller 26 (FIG. 3) described in the first embodiment described above, and further, step S3A is adopted instead of step S3, as illustrated in FIG. 9. Therefore, only steps S10 and S3A will be described in the following.

Step S10 is executed after step S1.

To be specific, in step S10, the detection region setting section 264a determines an area including pixel positions of the subject distances included in the attention range in the entire image area of the captured image acquired in step S1, on the basis of the depth map information acquired in step S1 and the attention range stored in the storage unit 265 in step S9.

After step S10, the detection region setting section 264a sets the area determined in step S10 as the detection region (step S3A). After that, the processing proceeds to step S4, and the detection process is executed in the detection region.

For example, as illustrated in FIGS. 10 to 12, it is assumed that, at the time of surgical operation, an obstacle EX such as an operator's hand or a surgical instrument enters the space between the imaging unit 21 and the observation target OB, and the obstacle EX is included in the captured image obtained by the imaging unit 21. In this case, since the obstacle EX is located outside the range RG corresponding to the attention range (represented by diagonal lines in FIGS. 11 and 12), the area other than the area in which the obstacle EX is included in the entire image area of the captured image is set as the detection region.

According to the second embodiment described above, in addition to a similar effect to that of the first embodiment described above, the following effects are exhibited.

The controller 26 according to the second embodiment determines the attention range in advance by the pre-operation. Further, when the controller 26 executes the main operation, an area having pixel positions of the subject distances included in the attention range in the entire image area of the captured image is set as the detection region. Then, the controller 26 executes the AF process and the brightness adjustment process on the basis of the detection information obtained by the detection process in the detection region.

Accordingly, even in a case where the obstacle EX is included in the captured image, the obstacle EX is not focused, and the brightness is not adjusted according to the obstacle EX. Therefore, according to the second embodiment, an image suitable for observation can be generated.

### (Third Embodiment)

Next, the third embodiment will be described.

In the following description, similar components to those in the first embodiment will be designated by the same reference numerals, and detailed description thereof will be omitted or simplified.

In the third embodiment, the operation of the controller 26 is different from that of the first embodiment described above.

Hereinafter, the operation of the controller 26 will be described.

FIG. 13 is a flowchart illustrating the operation of the controller 26 according to the third embodiment. FIGS. 14 and 15 are diagrams illustrating the operation of the controller 26.

Note that the observation target OB illustrated in FIGS. 14 and 15 is the same as the observation target OB illustrated in FIGS. 4 to 6.

In the operation of the controller 26 according to the third embodiment, as illustrated in FIG. 13, in the operation of the controller 26 (FIG. 3) described in the above first embodiment, step S11 and S12 are adopted instead of step S2, and further step S3B is adopted instead of step S3 with step S6 omitted. Therefore, only steps S11, S12, and S3B will be described below.

Step S11 is executed after step S1.

To be specific, the detection region setting section 264a acquires the current focal position detected by the focal position detection unit 214a via the communication unit 261 in step S11.

After step S11, the detection region setting section 264a determines an area currently observed by an operator or the like (hereinafter referred to as an observed area) in the entire image area of the captured image obtained in step S1 on the basis of the depth map information acquired in step S1 and the current focal position acquired in step S11 (step S12).

To be specific, in step S12, the detection region setting section 264a converts the current focal position acquired in step S11 into the subject distance. Then, the detection region setting section 264a determines the area having the pixel positions of the converted subject distance in the entire image area of the captured image obtained in step S1 as the observed area on the basis of the depth map information acquired in step S1 and the converted subject distance.

For example, as illustrated in FIG. 14(a), it is assumed that the current focal position is deep and the region observed by the operator or the like is the deepest area Ar. In this case, the area (observed area) having the pixel positions of the subject distance converted from the focal position is determined to be the region corresponding to the deepest area Ar as illustrated by diagonal lines in FIG. 14(b).

Further, for example, as illustrated in FIG. 15(a), in a case where the current focal position is shallow and the region observed by the operator or the like is the surface OB1, the region (observed area) having the pixels of the subject distance converted from the focal position is determined to be a region corresponding to the surface OB1 as illustrated by diagonal lines in FIG. 15(b).

After step S12, the detection region setting section 264a sets the observed area determined in step S12 as the detection region (step S3B). After that, the processing proceeds to step S4, and the detection process is executed in the detection region.

Further, in the second embodiment, the step S7 is executed after the step S5 because the step S6 is omitted.

According to the third embodiment described above, the following effects are exhibited in addition to a similar effect to that of the first embodiment described above.

The controller 26 according to the third embodiment determines the observed area in the captured image on the basis of the depth map information and the current focal position and sets the observed area as the detection region. Then, the controller 26 executes the brightness adjustment process on the basis of the detection information obtained in the detection process in the detection region.

Due to this, the brightness of the image corresponding to the area observed by the operator is automatically adjusted to a desired brightness in the captured image. Accordingly, an image suitable for observation can be generated according to the third embodiment.

### (Modification Example of Third Embodiment)

FIG. 16 illustrates diagrams explaining a modification example of the third embodiment.

Note that an observation target OB' illustrated in FIG. 16 is different from the observation target OB illustrated in FIGS. 14 and 15 in that the observation target OB' is further provided with a recess OB3 in which the depth position of the deepest area Ar' is the same as that of the area Ar.

In the third embodiment described above, as illustrated in FIG. 16(a), it is assumed that the current focal position is deep, and the subject distance converted from the focal position is the same as the subject distance at the pixel positions of the areas Ar and Ar'. In this case, the detection region setting section 264a cannot understand whether to set the region corresponding to the area Ar or the region corresponding to the area Ar' as the detection region in the entire image area of the captured image.

Further, it is assumed that the detection region in the entire image area of the captured image can be selected according to the user operation by the operator on a mouse, a keyboard, or an operation device (not illustrated) such as a touch panel provided on the controller 26, and a region corresponding to the area Ar is included in the selected detection region (in a case where a region corresponding to the area Ar' is not included in the detection region). In this case, the detection region setting section 264a sets the region corresponding to the area Ar as the detection region, between the area corresponding to the area Ar and the area corresponding to the area Ar' in the entire image area of the captured image in consideration of the detection region selected by the operator, as illustrated by the diagonal lines in FIG. 16(b).

### (Fourth Embodiment)

Next, the fourth embodiment will be described.

In the following description, similar components to those in the third embodiment will be designated by the same reference numerals, and detailed description thereof will be omitted or simplified.

In the fourth embodiment, the operation of the controller 26 is different from that of the third embodiment described above.

Hereinafter, the operation of the controller 26 will be described.

FIG. 17 is a flowchart illustrating the operation of the controller 26 according to the fourth embodiment. FIG. 18 illustrates diagrams explaining the operation of the controller 26.

Note that the observation target OB illustrated in FIG. 18 is the same observation target as that illustrated in FIGS. 4 to 6.

Regarding the operation of the controller 26 according to the fourth embodiment, as illustrated in FIG. 17, steps S13 and S14 are adopted instead of steps S3B and S4 to S7 in the operation of the controller 26 described in the above third embodiment (FIG. 13). Therefore, only steps S13 and S14 will be described below.

Step S13 is executed after step S12.

To be specific, in step S13, the operation control section 264c determines whether or not the subject distance of the observed area determined in step S12 is equal to or greater than a specific threshold value.

For example, as illustrated in FIG. 18(a), it is assumed that the current focal position is deep and the area observed by the operator or the like is deeper than the surface OB1 (indicated by diagonal lines in FIG. 18(b)). In this case, the result is determined to be "Yes" in step S13.

In a case where the result is determined to be "Yes" in step S13, the operation control section 264c adjusts the depth of field (step S14).

To be specific, the operation control section 264c increases the aperture value and the depth of field by controlling the operation of the aperture drive unit 213b in step S14. Alternatively, the operation control section 264c controls the operation of the image processing unit 262 in step S14 to cause the image processing unit 262 to perform image processing on the captured image acquired in step S1 for increasing the depth of field. Note that a known method can be employed for image processing for increasing the depth of field.

On the other hand, in a case where the result is determined to be "No" in step S13, the operation control section 264c ends the control flow without executing step S14.

According to the fourth embodiment described above, the following effects are exhibited in addition to a similar effect to that of the first embodiment described above.

Incidentally, in a case where the surgical operator is performing the operation of the deepest area Ar of the observation target OB, the operator wants to observe regions of other depths, too (for example, the surface OB1).

The controller 26 according to the fourth embodiment determines the observed area in the captured image on the basis of the depth map information and the current focal position, and controls to increase the depth of field in a case where the subject distance of the observed area is equal to or more than a specific threshold value. That is, in a case where the operation of the deepest area Ar is being performed, the captured image is an image in which the surface OB1 is in focus in addition to the deepest area Ar. Accordingly, an image suitable for observation can be generated according to the fourth embodiment.

### (Fifth Embodiment)

Next, the fifth embodiment will be described.

In the following description, similar components to those in the first embodiment will be designated by the same reference numerals, and detailed description thereof will be omitted or simplified.

In the first embodiment described above, the present disclosure is applied to the medical observation system 1 using the surgical operation microscope (medical observation device 2).

On the other hand, in the fifth embodiment, the present disclosure is applied to a medical observation system using a rigid endoscope.

FIG. 19 is a diagram illustrating a medical observation system 1D according to the fifth embodiment.

As illustrated in FIG. 19, the medical observation system 1D according to the fifth embodiment includes a rigid endoscope 2D, a light source unit 24 that is connected to the rigid endoscope 2D via a light guide 25 and that generates the illumination light emitted from the tip of the rigid endoscope 2D, a controller 26 that processes the image signal output from the rigid endoscope 2D, and a display device 3 for displaying a captured image based on the video signal for display processed by the controller 26.

As illustrated in FIG. 19, the rigid endoscope 2D includes an insertion portion 4 and a camera head 21D.

The insertion portion 4 has an elongated shape and is totally hard, or partially soft with the other part hard, and is inserted into the living organism. Then, the insertion portion 4 takes in light (subject image) from the living organism.

The camera head 21D is detachably connected to the base end (eyepiece) of the insertion portion 4. The camera head 21D has a substantially similar configuration to the imaging unit 21 described in the above-mentioned first embodiment. Then, the camera head 21D captures the subject image taken in by the insertion portion 4 and outputs an image signal.

Even in a case where the rigid endoscope 2D is used as in the fifth embodiment described above, a similar effect to that of the first embodiment described above can be obtained.

### (Sixth Embodiment)

Next, the sixth embodiment will be described.

In the following description, similar components to those in the first embodiment will be designated by the same reference numerals, and detailed description thereof will be omitted or simplified.

In the first embodiment described above, the present disclosure is applied to the medical observation system 1 using the surgical operation microscope (medical observation device 2).

On the other hand, in the sixth embodiment, the present disclosure is applied to a medical observation system using a flexible endoscope.

FIG. 20 is a diagram illustrating a medical observation system 1E according to the sixth embodiment.

As illustrated in FIG. 20, the medical observation system 1E according to the sixth embodiment includes a flexible endoscope 2E that captures an in-vivo image of the observation site by inserting an insertion portion 4E into a living organism and outputs an image signal, a light source unit 24 that generates illumination light emitted from the tip of the flexible endoscope 2E, a controller 26 that processes an image signal output from the flexible endoscope 2E, and a display device 3 for displaying a captured image based on a video signal for display processed by the controller 26.

As illustrated in FIG. 20, the flexible endoscope 2E includes the flexible and elongated insertion portion 4E, an operation unit 5 connected to the base end side of the insertion portion 4E and accepting various operations, and a universal cord 6 extends from the operation unit 5 in a direction different from the extending direction of the insertion portion 4E and containing various cables connected to the light source unit 24 and the controller 26.

As illustrated in FIG. 20, the insertion portion 4E includes a tip 41, a bendable curvature portion 42 connected to the base end side of the tip 41 and having a plurality of bend pieces, and a long flexible tube 43 connected to the base end side of the curvature portion 42 and having flexibility.

Then, although a specific illustration is omitted, a configuration substantially similar to that of the imaging unit 21 described in the first embodiment mentioned above is built in the tip 41. Then, the image signal from the tip 41 is output to the controller 26 via the operation unit 5 and the universal cord 6.

Even in a case where the flexible endoscope 2E is used as in the sixth embodiment described above, a similar effect to that of the first embodiment described above can be obtained.

### (Other Embodiments)

Although the embodiments for carrying out the present disclosure have been described so far, the present disclosure should not be limited only to the above-described embodiments.

FIG. 21 is a diagram illustrating a modification example of the first to fourth embodiments.

In the above-described first to fourth embodiments, the distance information acquiring unit according to the present disclosure integrally includes the image pickup device 215 (imaging unit 21), but the present invention is not limited to this, and the distance information acquiring unit may be separated from the imaging unit 21. In such a configuration, as illustrated in FIG. 21, the visual field area Ar1 whose image is captured by the imaging unit 21 and the depth map acquisition area Ar2 from which the distance information acquiring unit according to the present disclosure acquires depth map information are different. Therefore, as the depth map information, information that limits the depth map acquisition area Ar2 to the visual field area Ar1 is used on the basis of the focal position, the angle of view, and the like in the imaging unit 21.

Note that although the first to fourth embodiments have been described in FIG. 21, the distance information acquiring unit according to the present disclosure may be configured separately from the imaging unit provided on the camera head 21D and the tip 41 also in the fifth and sixth embodiments.

In the above-described first to sixth embodiments, it is sufficient if the brightness adjustment process includes an adjusting process for only some of the exposure time of each pixel in the image pickup device 215, the analog gain to be multiplied by the signal processing unit 216, the digital gain to be multiplied by the image processing unit 262, and the amount of illumination light supplied by the light source unit 24.

In the medical observation device 2 according to the above-described first to fourth embodiments, the first to sixth axes O1 to O6 are respectively configured by passive axes, but the present invention is not limited to this. It is sufficient if at least one of the first to sixth axes O1 to O6 is configured by an active axis that actively rotates the imaging unit 21 around the axis according to the power of the actuator.

In the above-described first to third embodiments, it is sufficient if a configuration is adopted in which the detection region is displayed on the display device 3 or the like in order to indicate, to the operator and the like, what is the range of the detection region set in steps S3, S3A, and S3B.

In the above-described first to sixth embodiments, it is sufficient if the order of processing in the operation flows illustrated in FIGS. 3, 7, 9, 13, and 17 is changed within a range of consistency. In addition, it is sufficient if the techniques described in the above-described first to sixth embodiments are combined as appropriate.

### [Reference Signs List]

1, 1D, 1E: Medical observation system
2: Medical observation device
2D: Rigid endoscope
2E: Flexible endoscope
3: Display device
4, 4E: Insertion portion
5: Operation unit
6: Universal cord
21: Imaging unit
21D: Camera head
22: Base
23: Support
24: Light source unit
25: light guide
26: Controller
41: Tip
42: Curvature portion
43: Flexible tube
211: Lens unit
211a: Focus lens
212: Aperture
213: Drive unit
213a: Lens drive unit
213b: Aperture drive unit
214: Detection unit
214a: Focal position detection unit
214b: Aperture value detection unit
215: Image pickup device
216: Signal processing unit
217: Communication unit
218: Focus switch
221: Caster
231a: First arm
231b: Second arm
231c: Third arm
231d: Fourth arm
231e: Fifth arm
231f: Sixth arm
231g: Seventh arm
232a: First joint
232b: Second joint
232c: Third joint
232d: Fourth joint
232e: Fifth joint
232f: Sixth joint
233: Counterweight
261: Communication unit
262: Image processing unit
263: Display control unit
264: Control unit
264a: Detection region setting section
264b: Evaluation value calculating section
264c: Operation control section
265: Storage unit
Ar, Ar': Deepest area
Ar1: Visual field area
Ar2: Depth map acquisition area
EX: Obstacle
O1: First axis
O2: Second axis
O3: Third axis
O4: Fourth axis
O5: Fifth axis
O6: Sixth axis
OB, OB': Observation target
OB1: Surface
OB2, OB3: Recess
RG: Range

## Claims

1. A medical observation system comprising:
an imaging unit configured to capture an image of a subject and generates a captured image;
a distance information acquiring unit configured to acquire subject distance information regarding a subject distance from a specific position to a corresponding position on the subject, the corresponding position corresponding to each of at least two pixel positions in the captured image; and
an operation control section configured to control at least any of a focal position of the imaging unit, a brightness of the captured image, and a depth of field of the imaging unit, on a basis of the subject distance information.

2. The medical observation system according to claim 1, wherein
the subject distance information includes depth map information obtained by detecting the subject distance from the specific position to a corresponding position on the subject, the corresponding position corresponding to a pixel position in the captured image, for each of the pixel positions.

3. The medical observation system according to claim 1 or 2, wherein
the operation control section determines a pixel position of the subject distance that is specific in the captured image, on the basis of the subject distance information, and controls the focal position of the imaging unit such that an area including the determined pixel position is in focus.

4. The medical observation system according to claim 3, wherein
the pixel position of the specific subject distance is a pixel position whose subject distance is the largest in the captured image.

5. The medical observation system according to claim 3 or 4, wherein
the operation control section determines the pixel position of the specific subject distance in the captured image among the pixel positions in a central region including a center of the captured image, on the basis of the subject distance information, and controls the focal position of the imaging unit such that the area including the determined pixel position is in focus.

6. The medical observation system according to any one of claims 1 to 5, the system further comprising:
a detection region setting section configured to set a detection region in the captured image; and
an evaluation value calculating section configured to calculate an evaluation value used for at least one of control of the focal position of the imaging unit and control of the brightness of the captured image executed by the operation control section, on a basis of the image in the detection region in the captured image, wherein
the detection region setting section determines a range of the subject distance to pay attention to, on the basis of the subject distance information, and sets an area including a pixel position of the subject distance included in the determined range of the subject distance in the captured image as the detection region.

7. The medical observation system according to any one of claims 1 to 6, the system further comprising:
a focal position detecting unit configured to detect a current focal position in the imaging unit, wherein
the operation control section adjusts the brightness of the captured image, on a basis of the subject distance information and the current focal position.

8. The medical observation system according to claim 7, the system further comprising:
a detection region setting section configured to set a detection region in the captured image; and
an evaluation value calculating section configured to calculate an evaluation value used for controlling the brightness of the captured image by the operation control section, on the basis of the image in the detection region in the captured image, wherein
the detection region setting section determines a currently observed region in the captured image, on the basis of the subject distance information and the current focal position, and sets the determined region as the detection region.

9. The medical observation system according to any one of claims 1 to 8, the system further comprising:
a focal position detecting unit configured to detect a current focal position in the imaging unit, wherein
the operation control section controls the depth of field of the imaging unit, on the basis of the subject distance information and the current focal position.

10. The medical observation system according to claim 9, wherein
an imaging unit includes an image pickup device that receives light from the subject and generates the captured image, and
an aperture provided between the subject and the image pickup device and adjusting an amount of light incident on the image pickup device from the subject, and
the operation control section controls the depth of field of the imaging unit by controlling an operation of the aperture.

11. The medical observation system according to claim 9, the system further comprising:
an image processing unit configured to execute image processing on the captured image to adjust the depth of field, wherein
the operation control section controls the depth of field of the imaging unit by controlling an operation of the image processing unit.

12. The medical observation system according to any one of claims 9 to 11, wherein
the operation control section determines the currently observed pixel position in the captured image, on the basis of the subject distance information and the current focal position, and performs control such that the depth of field of the imaging unit is increased in a case where the subject distance at the determined pixel position is equal to or greater than a specific threshold value.

13. The medical observation system according to any one of claims 1 to 12, wherein
the distance information acquiring unit includes any of a phase difference sensor, a TOF sensor, and a stereo camera.

14. The medical observation system according to claim 13, wherein
the distance information acquiring unit is provided in the imaging unit, acquires the subject distance information, and generates the captured image.
